# FASCICULE DE BREVET EUROPEEN

(11) **EP 2 628 321 B1**
(45) Date de publication et mention de la délivrance du brevet: **11.04.2018**
(21) Numéro de dépôt: 11832112.4
(22) Date de dépôt: 13.10.2011
(51) Int. Cl.: H04R 25/00, A61L 2/18, B08B 3/00, B08B 7/00, B08B 3/04, B08B 3/10

(54) **DISPOSITIF DE NETTOYAGE D'UNE PROTHESE AUDITIVE**
VORRICHTUNG ZUR REINIGUNG EINES HÖRGERÄTS
DEVICE FOR CLEANING A HEARING AID

(30) Priorité: 13.10.2010 FR 1058349
(43) Date de publication de la demande: 21.08.2013
(73) Titulaire: MG Developpement, 34470 Perols (FR)
(72) Inventeur: GIL, José Antonio, 34160 St Bauzille De Montmel (FR)
(74) Mandataire: Rhein, Alain
(86) Numéro de dépôt international: PCT/FR2011/052389
(87) Numéro de publication internationale: WO 2012/049429

(56) Documents cités:
- WO-A1-2005/125276
- FR-A1- 2 922 770
- US-A1- 2008 128 007

## Description

La présente invention entre dans le domaine médical de l'audition, en particulier des appareillages médicaux auditifs pour les personnes atteintes de surdités et malentendantes.

De manière connue, de tels appareillages se présentent généralement sous la forme d'une prothèse auditive venant se positionner, de manière cachée et quasi-invisible, derrière l'oreille ou dans le conduit auditif. Cette prothèse inclut des moyens d'alimentation sous forme d'une pile ou batterie, ainsi que des moyens de traitement des sons perçus et de transmission vers des moyens d'émission auxquels elle est reliée. Ces moyens d'émission se présentent sous la forme d'un écouteur conformé, qui peut être réalisé sur mesure, de manière à être introduit à l'intérieur du conduit auditif. Ledit écouteur permet d'émettre les sons traités et transmis depuis ladite prothèse, souvent en les amplifiant.

De telles prothèses auditives, du fait de l'insertion de l'écouteur au sein du conduit auditif, améliorent considérablement l'écoute des personnes appareillées. Toutefois, elles présentent un inconvénient majeur lié au contact direct dudit écouteur avec le cérumen, mais aussi avec toutes les poussières, saletés et particules de matière qu'il véhicule le long et vers l'extérieur du canal auditif. Il en résulte une diminution de l'efficacité de l'écouteur et une dégradation de l'audition de la personne appareillée.

Plus contraignant, certaines prothèses sont détériorées et renvoyées auprès du fabriquant pour être réparée ou remplacée, générant un coût et des délais contraignants.

C'est pourquoi il convient de nettoyer régulièrement l'écouteur afin de le débarrasser desdites impuretés. Mais les composants électroniques intégrés au sein de l'écouteur interdisent l'utilisation de liquide pour ce nettoyage.

Une solution connue est décrite dans le document WO 2005/12576 relatif à un dispositif de diagnostique et de nettoyage d'une prothèse auditive, en particulier de son écouteur. Un tel dispositif permet à un particulier d'effectuer à domicile un diagnostique de son appareillage et de le nettoyer périodiquement, sans recourir à un quelconque liquide susceptible de détériorer les composants électroniques constituant ledit écouteur. Un tel nettoyage s'effectue par séchage du cérumen et aspiration des particules ainsi desséchées.

Pour ce faire, des moyens de chauffage sont disposés à l'intérieur d'un logement de réception dudit écouteur de manière à diffuser une chaleur suffisante au sein de cet espace pour dessécher le cérumen. Ces moyens de chauffage se présentent sous la forme de résistances électriques ou bien de sources de rayonnement ultraviolet. Un ventilateur est ajouté afin d'assurer une circulation de l'air ainsi chauffé, notamment au sein de l'écouteur. Dès lors, le taux d'humidité présent dans le cérumen est diminué jusqu'à permettre l'aspiration des résidus desséchés par une pompe à vide et leur évacuation vers un réservoir de stockage.

Un tel dispositif de nettoyage présente un inconvénient majeur lié au séchage du cérumen. En effet, si la plupart des particules desséchées sont aspirées par la pompe à vide, une partie des résidus demeure, souvent coincés dans les anfractuosités de l'écouteur.

Un dispositif similaire est décrit dans le document WO 98/4885, utilisant une mise en circulation d'air chaud par soufflage et aspiration.

Une autre solution a été imaginée au travers du document WO 2007/005748 qui décrit un dispositif de nettoyage de prothèses auditives par centrifugation. En somme, lesdites prothèses sont placées dans un compartiment entraîné en rotation jusqu'à atteindre une vitesse de manière à soumettre lesdites prothèses à une force de l'ordre de 200 à 400 G. A cette vitesse, les particules de cérumen sont éjectées et aspirées. Des ondes ultrasonores peuvent aussi faciliter le détachement des particules de cérumen lors de la centrifugation. Il est aussi possible de chauffer l'enceinte, entre 41 et 60 degrés Celsius, de manière à dessécher le cérumen et encore une fois faciliter son détachement.

Selon un mode de réalisation spécifique, la prothèse est plongée dans un liquide nettoyage, mis aussi en rotation, puis séchée par une étape supplémentaire de centrifugation. Toutefois, l'électronique contenue dans la prothèse est très sensible à l'humidité, de sorte que l'immersion dans un liquide peut provoquer une détérioration.

Une autre solution est connue du document US 2008/128007 qui décrit un dispositif de nettoyage et de rechargement d'une prothèse auditive. En particulier, ce nettoyage s'effectue par l'intermédiaire d'un fluide, mis en circulation depuis une chambre, jusqu'à être mis en contact avec ledit écouteur.

A ce titre, l'écouteur est positionné dans un logement, de forme complémentaire et dont la paroi interne assure un « grip » pour le maintenir en position. De plus, positionné au sein dudit logement, l'écouteur est disposé de sorte qu'une portion dudit écouteur s'étende vers le bas au travers de la paroi du logement. Une fois positionné, le fluide de nettoyage remplit la cavité située sous le logement, pour n'immerger que ladite portion qui dépasse.

Si une telle solution permet de cibler le nettoyage au niveau de l'extrémité de l'embout de l'écouteur, aucune évacuation ou aspiration du fluide n'est prévue dans la cavité inférieure pendant le nettoyage, laissant l'embout immergé, ce qui résulte d'un nettoyage peu satisfaisant, plus long du fait de la simple immersion et susceptible d'endommager l'électronique.

L'invention a pour but de pallier les inconvénients de l'état de la technique en proposant un dispositif de nettoyage d'un appareillage médical auditif, en particulier de son écouteur, constitué d'un embout conformé de manière à être introduit dans le conduit auditif d'une personne, au travers de la liquéfaction du cérumen, puis de son aspiration.

Pour ce faire, un tel dispositif de nettoyage prévoit d'humidifier le cérumen pour permettre sa liquéfaction totale.

Avantageusement, l'humidification selon l'invention est localisée au niveau des embouts conformés de manière à être introduits à l'intérieur du conduit auditif, sans entrer en contact avec le reste de la prothèse. En somme, un liquide vient lécher ces embouts, sans que ces derniers restent en contact immergés et, par conséquent, sans risque de détériorer l'électronique de l'écouteur et de ladite prothèse.

Pour ce faire, le dispositif de nettoyage selon l'invention comprend, d'une part, un espace de réception de l'écouteur qui comprend au moins un logement conformé de manière à recevoir l'embout, le logement comprenant en partie basse un orifice conformé de sorte que l'extrémité distale de l'embout dépasse au sein d'une cavité ménagée sous le logement, et d'autre part, des moyens de mise en circulation au sein de l'espace d'un liquide de sorte que ce dernier vienne au contact de l'embout, la cavité comprenant au moins une sortie d'écoulement ou d'aspiration du liquide, débouchant en vis-à-vis sous chaque orifice, caractérisé en ce que les moyens de mise en circulation projettent le liquide, afin que le liquide vienne effleurer l'extrémité distale de l'embout, le fond de la cavité comprend des moyens d'orientation du liquide jusqu'à la sortie située en dessous de l'orifice, l'évacuation et l'écoulement ou l'aspiration assurant que la cavité ne se remplisse pas de fluide.

Un autre avantage de la présente invention réside dans la possibilité d'utiliser un liquide désinfectant.

Dès lors, le chauffage introduit dans l'invention n'a pour unique but que de sécher la partie de la prothèse qui a été nettoyée par humidification.

Selon d'autres caractéristiques additionnelles, correspondant à des modes de réalisations particuliers, lesdits moyens d'orientation se présentent sous la forme de saillies, de striures ou de gorges.

Préférentiellement, ledit dispositif comprend des moyens de chauffage dudit espace par mise en circulation d'air chauffé.

En particulier, un tel dispositif peut comprendre des moyens de filtration situés en partie inférieure sous ladite cavité.

Par ailleurs, lesdits moyens de filtration se présentent sous la forme d'une membrane perméable constituée en fibres, mousse ou en un matériau tissé dont l'espacement permet de récupérer les particules de cérumen, liquéfiée ou non, ainsi que les poussières et saletés, tout en laissant passer le liquide.

Selon un mode spécifique de réalisation, le dispositif comprend une cartouche de forme cylindrique insérée au sein dudit espace, ladite cartouche étant subdivisée intérieurement en plusieurs compartiments de stockage et de recyclage délimités par la paroi périphérique dudit cylindre et des parois radiales.

Selon un mode particulier de réalisation, lesdits compartiments de stockage comprennent des orifices ménagés en face inférieure refermés par des opercules, ainsi que des trous ménagés en face supérieure, et que lesdits compartiments de recyclage comprennent deux orifices de recyclage ménagés en face supérieure de ladite cartouche.

Préférentiellement, ladite cartouche comprend deux canaux centraux sous forme cylindrique traversant intérieurement ledit cylindre depuis la face supérieure jusqu'à la face inférieure et débouchant à l'extérieur au niveau réciproquement de trous supérieurs ménagés en face supérieure et de trous inférieurs ménagés en face inférieure.

En particulier, ledit dispositif comprend un dôme présentant une section complémentaire à celle de la section de l'espace, de manière à venir s'y insérer en partie supérieure et, après insertion, ledit dôme étant monté mobile est rotation selon l'axe central dudit espace, de manière à être tourné tourner dans le sens des aiguilles d'une montre, ou inversement, dans plusieurs positions de fonctionnement.

De manière additionnelle, ledit dôme comprend une partie supérieure et une partie inférieure, cette dernière étant creusée, de manière à y ménager des rainures à l'intérieure desquelles circulera le fluide lors du nettoyage, une ouverture étant ménagée en périphérie et reliée à une bifurcation reliant deux rainures symétriques venant déboucher chacune au droit d'une paroi en arc de cercle déviant de part et d'autre ledit fluide lors de sa circulation jusqu'au niveau de chaque extrémité de chaque paroi vers une butée ménagée à angle droit détournant le fluide vers l'intérieur.

D'autres caractéristiques et avantages de l'invention ressortiront de la description détaillée qui va suivre des modes de réalisation non limitatifs de l'invention, en référence aux figures annexées dans lesquelles :
- la figure 1 représente une vue en perspective du dispositif de nettoyage en position d'ouverture, avec deux prothèses placées à l'intérieur ;
- la figure 2 est une vue en perspective éclatée d'un détail dudit dispositif avec deux prothèses ;
- la figure 3 est une vue à plat de dessus ;
- la figure 4 est une vue en coupe selon le plan vertical A-A' de la figure 3 ;
- la figure 5 est une vue en coupe selon le plan horizontal B-B' de la figure 4 ;
- la figure 6 est une vue en coupe selon le plan horizontal C-C' de la figure 4 ;
- la figure 7 est une représentation éclatée en perspective d'une cartouche de rechargement du dispositif selon l'invention ;
- la figure 8 est une vue en perspective selon un plan de coupe horizontal d'une telle cartouche ; et
- la figure 9 est une vue en perspective éclatée d'un autre détail de l'invention.

La présente invention concerne le nettoyage d'un appareillage médical auditif 1, notamment une prothèse auditive.

Une telle prothèse comprend un boîtier 100 conformé de manière à venir se placer derrière l'oreille et relié de manière filaire 102, ou non, à un écouteur 101 sous forme d'un embout ou « dôme » conformé de manière à s'insérer au sein du conduit auditif de la personne appareillée. Un tel embout peut être un écouteur 101 nu dont l'extrémité est recouverte d'une collerette standard pour l'insertion et le maintien au sein du conduit auditif, ou bien directement conformé sur mesure.

Avantageusement, l'invention a pour objet un dispositif 2 de nettoyage d'un tel appareillage médical auditif 1, en particulier de l'embout constituant son écouteur 101.

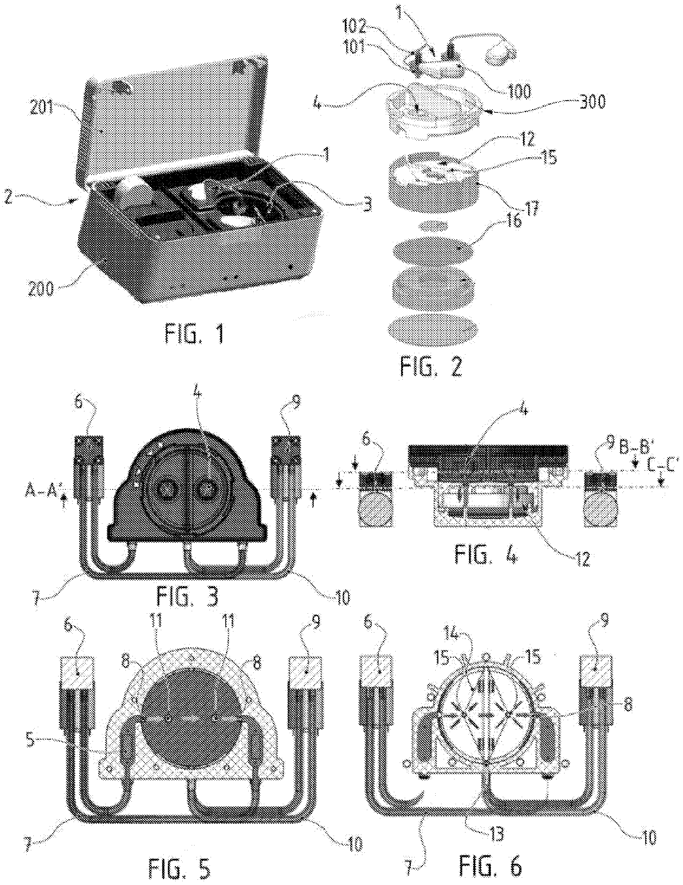

Pour ce faire, comme visible sur la figure 1, le dispositif de nettoyage 1 selon l'invention comprend une enceinte sous forme d'une boîte 200 sur laquelle est monté articulé, par l'intermédiaire de charnières, un couvercle 201, de manière à passer d'une position d'ouverture à une position fermée, et inversement.

On notera qu'en position refermée, ladite boîte 200 peut être hermétique, notamment étanche à l'air et/ou à tout fluide ou liquide.

Une telle enceinte comprend un espace 3 de réception dudit écouteur 101.

En particulier, ledit espace 3 est conformé de manière à permettre l'insertion totale ou partielle dudit écouteur 101.

Selon un mode préférentiel de réalisation, ledit espace 3 comprend au moins un logement 4 au sein duquel peut être placé ledit écouteur 101. En particulier, ledit logement 4 est ménagé au sein d'une plaque fermoir 300. Ledit espace 3, en particulier cette plaque 300, peut aussi préférentiellement comprendre deux logements 4, chacun destiné à recevoir chaque embout des écouteurs 101 de deux prothèses 1. Ladite plaque 300 sert alors de support de « dôme ».

Chaque logement 4 est conformé complémentairement audit écouteur 101 ou bien est ménagé au sein d'un matériau souple et élastique, tel par exemple du silicone ou un matériau alvéolaire souple ou semi-rigide, possédant des caractéristiques élastiques ou une certaine résilience, de type mousse, permettant sa déformation lors de l'insertion dudit écouteur 101, son maintien au sein du logement 4, ainsi que son retour en forme après extraction de l'embout de l'écouteur 101.

Le nettoyage dudit écouteur 101 s'effectue par liquéfaction du cérumen, des résidus, poussières ou analogue.

Avantageusement, une caractéristique essentielle de la présente invention réside dans l'humidification de l'embout de chaque écouteur 101 au cours de son nettoyage, afin de permettre la liquéfaction du cérumen et le retrait total de ce dernier.

Plus particulièrement, l'humidification de l'embout s'opère au travers de la mise en circulation d'un fluide, ledit fluide venant au contact dudit embout.

On notera que ledit fluide peut être un liquide de type solution, notamment un liquide de nettoyage et/ou désinfectant,

De plus, ledit dispositif comprend des moyens 9 de mise en circulation dudit fluide au sein d'un circuit 10. Lesdits moyens 9 permettent l'injection et l'aspiration dudit fluide au travers dudit circuit 10 et réciproquement vers et depuis ledit espace 3.

En particulier, l'injection et l'aspiration dudit fluide s'effectuent au niveau de chaque logement 4, de manière à venir effleurer une partie de l'embout de chaque écouteur 101.

Comme visible sur le mode de réalisation représenté sur les figures, chaque logement 4 comprend en sa partie inférieure au moins un orifice 11 à l'intérieur duquel vient s'insérer l'extrémité de l'embout d'un écouteur 101. Un tel orifice 11 permet donc de laisser dépasser sous le logement 4 l'extrémité distale dudit embout destinée à venir s'introduire au plus profond au sein du conduit auditif, et par conséquent étant la partie la plus sale à nettoyer.

La circulation du liquide de nettoyage s'effectue en partie sous-jacente desdits logements 4. En particulier, une cavité 12 est ménagée au sein dudit espace 3, sous le logement 4. Cette cavité 12 est prévue étanche et permet l'injection du liquide et son écoulement.

En particulier, ledit liquide est projeté par l'intermédiaire d'au moins une buse 13 injectant le liquide au sein de ladite cavité 12. En somme, le circuit 10 est ouvert au niveau de la cavité 12. Dès lors, le liquide sort et s'écoule au niveau du fond de la cavité 12, conformé de manière à guider le liquide au contact de la partie de l'embout de chaque écouteur 101 dépassant par l'intermédiaire dudit orifice 11. Le liquide vient alors lécher cette extrémité, sans pour autant pénétrer dans la partie supérieure du logement 4 et de l'espace 3 situé supérieurement.

De préférence, ledit fond peut présenter des moyens 14 permettant d'orienter le liquide depuis chaque buse 13 vers le dessous orifices 11. De tels moyens d'orientation 14 peuvent se présenter sous forme de saillies, notamment des ailettes, ou sous forme de striures, gorges ou analogues.

Avantageusement, l'orientation, l'évacuation et l'aspiration assurent que la cavité ne remplisse pas de fluide, en formant une sorte de vortex, tel un tourbillon, sous l'extrémité distale de l'écouteur, au niveau de l'orifice correspondant de sortie. Le fluide circule alors pour uniquement nettoyer, sans rester à « stagner » au contact de l'embout.

Arrivé au niveau de chaque orifice 11, le liquide vient effleurer l'extrémité dudit embout inséré dans son logement 4, puis est aspiré par une sortie 15 débouchant en vis-à-vis de chaque orifice 11. L'écoulement ou l'aspiration au niveau de ladite sortie 15 renvoie alors le fluide à l'intérieur du circuit 10 vers les moyens de mise en circulation 9.

On notera que ce renvoi du fluide peut s'opérer directement à l'intérieur du circuit 10, vers des moyens de recyclage ou bien par l'intermédiaire de moyens de filtration 16 situés en partie inférieure, sous ladite cavité 12. En somme, les sorties 15 traversent donc la paroi inférieure de ladite cavité 12 de manière à guider le fluide vers lesdits moyens de filtration 16 situés en dessous.

De plus, comme visible sur la figure 2, lesdits moyens de filtration 16 peuvent se présenter sous la forme d'une membrane perméable, notamment constituée en fibres, mousse ou en un matériau tissé dont l'espacement permet de récupérer les particules de cérumen, liquéfiée ou non, ainsi que les poussières et saletés, tout en laissant passé le liquide. Ce dernier est alors redirigé vers les moyens de mise en circulation 9, où il peut être renvoyé dans le circuit 10.

Selon le mode de réalisation visible sur les figures, en particulier la figure 3, ledit espace 3 comprend en partie inférieure un réservoir 17 de stockage dudit liquide. Dès lors, le circuit 10 part dudit réservoir 17 pour aspirer le liquide jusqu'à une pompe constituant les moyens de mise en circulation 9 qui renvoie le liquide vers chaque buse 13. Le liquide s'écoule alors le fond de la cavité 12, le long des ailettes 14, jusqu'à la sortie 15, où il est filtré avant de revenir dans ledit réservoir 17.

La figure 6 schématise par des flèches la direction d'écoulement concentrique du fluide vers les deux sorties 15.

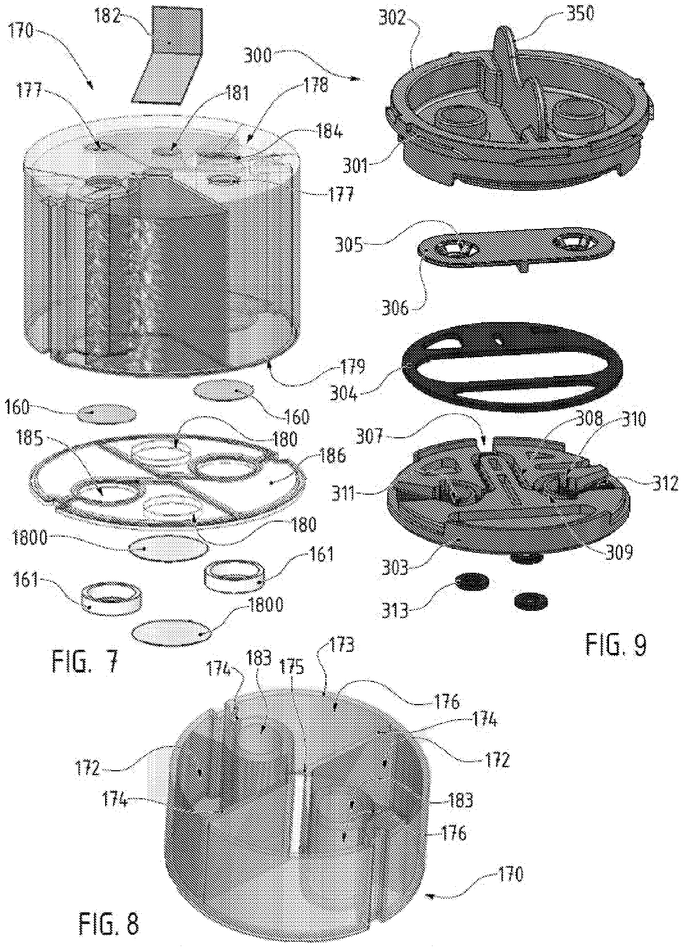

Selon un mode préférentiel de réalisation, ledit réservoir 17 peut être constituée par une cartouche amovible 170, telle que représentée sur les figures 7 et 8.

Ladite cartouche 170 se présente sous forme cylindrique, d'un diamètre égal ou inférieur au diamètre dudit espace 3, de manière à permettre son insertion avec ou sans jeu. De plus, la hauteur de la cartouche 170 est inférieure à la profondeur dudit espace 3, de manière à permettre son insertion totale à l'intérieur et autoriser le positionnement dudit support de dôme 300, assurant une fermeture étanche.

On notera que ledit espace 3 peut alors comprendre un ou plusieurs joints annulaires au niveau de sa paroi intérieure de manière à assurer l'étanchéité avec la paroi périphérique vertical de ladite cartouche 170, avec ledit support de dôme 300, et la partie inférieure dudit espace 3 qui constitue alors elle-même au moins en partie le réservoir 17.

Avantageusement, ladite cartouche 170 est subdivisée intérieurement en plusieurs compartiments.

En premier lieu, la cartouche 170 comprend des compartiments 172 de stockage dudit fluide de nettoyage. En somme, lorsque la cartouche 170 est utilisée pour la première fois, ces compartiments de stockage 172 sont fermés et remplis.

En particulier, la cartouche 170 comprend deux compartiments de stockage 172 se présentant sous la forme de portions du volume intérieur du cylindre constituant ladite cartouche 170. En somme, ces deux compartiments 172 sont chacun délimités par la paroi extérieure 173 périphérique et par deux parois sensiblement radiales ou radiales 174, ces dernières étant sécantes selon une arête 175 s'étendant parallèlement à l'axe central dudit cylindre, a proximité de ce dernier.

Ainsi, ces parois radiales 174 déterminent aussi deux autres compartiments 176 de recyclage du fluide après utilisation. Préférentiellement, ces deux compartiments de recyclage 176 se retrouvent reliés au niveau dudit axe central, formant une section en coupe horizontale assimilable à un sablier. Cette forme spécifique assure le remplissage du fluide recyclé à la même hauteur au sein de ces deux compartiments 176.

A ce titre, le remplissage de ces derniers s'effectue par deux orifices de recyclage 177 ménagés en face supérieure 178 de la cartouche 170.

Par ailleurs, en face inférieure 179, deux orifices 180 sont ménagés au niveau des compartiments de stockage 172. Ces orifices 180 sont refermés par des opercules 1800, destinés à être percés au moment de placer la cartouche 170 à l'intérieur de l'espace 3. Pour ce faire, des ergots de perforation peuvent être positionnés en saillie au fond dudit espace 3, de manière à venir trouer lesdits opercules 1800 et servir en même temps de détrompeur pour positionner parfaitement la cartouche 170 à l'intérieur de l'espace 3.

De plus, en face supérieure 178, des trous 181 sont ménagés au niveau desdits compartiments de stockage 172. Ces trous 181 sont refermés par une languette 182 prévu amovible ou perforable. Une fois retirée ou perforée, ladite languette 182 laisse rentrer l'air à l'intérieur de chaque compartiment de stockage 172 et le liquide peut alors s'écouler par les orifices 180, venant remplir le fond de l'espace 3, jusqu'à venir à niveau avec la cartouche 170.

Selon une caractéristique additionnelle, ladite cartouche 170 peut comprendre deux canaux centraux 183 sous forme cylindrique traversant intérieurement le cylindre depuis la face supérieure 178 jusqu'à la face inférieure 179 et débouchant à l'extérieur au niveau réciproquement de trous supérieurs 184 ménagés en face supérieure 178 et de trous inférieurs 185 ménagés en face inférieure 179.

Ces canaux 183 forment alors une partie du circuit par lequel transite le fluide au cours du nettoyage, depuis les logements 4 situés en vis-à-vis desdits trous supérieurs 184 lors de l'étape de nettoyage et de circulation du fluide.

Plus particulièrement, les trous inférieurs 185 peuvent présenter dans l'épaisseur de la paroi de la face inférieure 179 et/ou en partie dans l'épaisseur des parois desdits canaux 183, un élargissement pour recevoir les moyens de filtration 16. En particulier, ces derniers se présentent alors sous la forme de filtre circulaire 160. De tels filtres peuvent être maintenus en place par l'intermédiaire de bagues annulaires 161, venant s'insérer au sein desdits élargissement.

Par ailleurs, ces filtres 160 et ces bagues 161 peuvent être maintenus en place par l'intermédiaire d'une plaque de fond 186. Cette dernière peut être fixée au fond de la cartouche 170 de façon amovible ou non. Ladite plaque 186 présente aussi les orifices et trous précédemment évoqués pour permettre la circulation du fluide.

Avantageusement, ladite cartouche 170 peut présenter une symétrie par rapport à son axe central, facilitant son positionnement sans sens particulier, grâce auxdits détrompeurs formés par les saillies du fond de l'espace 3.

De ce qui précède, on comprend que le fluide peut circuler depuis les compartiments de stockage 172, au travers des canaux 183 ou vers les compartiments de recyclage 176.

Pour ce faire, ledit espace 3 est refermé après positionnement de la cartouche 170 par le support de dôme 300 de fermeture étanche. Ce dernier présente une section complémentaire à celle de la section de l'espace 3, de manière à venir s'y insérer en partie supérieure. Après insertion, ledit support de dôme 300 est mobile est rotation selon l'axe central dudit espace 3. Il est alors possible de le tourner dans le sens des aiguilles d'une montre ou inversement.

On notera que pour faciliter ces rotations, mais aussi l'insertion ou l'extraction, ledit support de dôme 300 peut comprendre des moyens de préhension 350, permettant à l'utilisateur de le prendre entre les doigts.

Tout d'abord, dans une première position, le support de dôme 300 peut être inséré ou retiré. En particulier, lors de son insertion, l'appui permet d'appuyer sur la cartouche 170 et de perforer les opercules 1800.

Dans une autre position, après une rotation selon un angle déterminé, le support de dôme 300 vient se bloquer en position de nettoyage. Dès lors, les logements 4 et les orifices 11 se retrouvent en vis-à-vis des trous supérieurs 184 de la cartouche 170, assurant la circulation du fluide pendant le nettoyage.

Dans une troisième position de recyclage, la rotation du support de dôme 300 selon un autre angle prédéfini, amène lesdits logements 4 et orifice 11 en vis-à-vis des orifices de recyclage 177 des compartiments de recyclage 176. Le système peut alors être purgé de tout son fluide.

On notera qu'en positions de nettoyage ou de recyclage, ledit support de dôme 300 est alors bloqué verticalement, à savoir qu'il ne peut être extrait, comme en première position.

Pour ce faire, le chant périphérique dudit support de dôme 300 peut présenter des rainures 301 destinées à coopérer avec des saillies complémentaires ménagées intérieurement en périphérie de la paroi haute dudit espace 3.

Selon le mode de réalisation préférentiel, ledit support de dôme 300 peut être constitué d'une partie supérieure 302 et d'une partie inférieure 303. Ces dernières sont alors fixées ensemble de manière amovible ou non, notamment par clipsage.

Entre ces deux parties 302 et 303, un joint 304 peut être positionné afin d'assurer l'étanchéité.

De plus, ledit support de dôme 300 peut comprendre une tige 305 servant de support à l'extrémité distale de l'embout des écouteurs 101 devant être nettoyés. En somme, cette tige support 305 assure le maintien de ladite extrémité au dessus du fluide lors du nettoyage, afin qu'elle ne plonge pas directement dedans et qu'elle soit uniquement « léchée » sans être immergée.

En particulier, ladite tige support 305 présente deux trous traversants chacun pourvu d'un « panier » 306 sur lequel vient reposer l'écouteur 101, en particulier l'embout standard ou sur mesure de ce dernier.

Cette tige 305 vient alors être prise en sandwich lors de la fixation des parties 302 et 303, une ouverture de forme complémentaire étant ménagée au niveau dudit joint 304. On notera alors que la tige 305 peut présenter une forme oblongue plate, approximativement ovale.

De plus, la partie inférieure 303 intègre une partie du circuit de nettoyage dudit fluide. En somme, cette partie inférieure 303 est creusée de manière à y ménager des rainures à l'intérieure desquelles circulera le fluide lors du nettoyage.

Selon le schéma préférentiel, mais aucunement limitatif, représenté sur la figure 9, une ouverture 307 est ménagée en périphérie. Elle est reliée à une bifurcation reliant deux rainures symétriques 308 venant déboucher chacune au droit d'une paroi 309 en arc de cercle, de préférence en demi-cercle. Lors de la circulation du fluide, il se retrouve dévié de part et d'autre. Toutefois, une quantité de fluide plus importante se dirige d'un côté de la paroi 309 (vers le bas sur la figure 9), en raison de l'orientation de la rainure 308. Puis, au niveau de chaque extrémité de chaque paroi 309, une butée 310 est ménagée à angle droit. Le fluide dévié par la paroi 309 se retrouve alors détourné vers l'intérieur.

On notera que dans l'espace circulaire de l'autre côté de la paroi 309, est ménagé une sortie 311 (coïncidant avec la sortie 15).

Alors, le fluide guidé par les éléments précédents, vient créer le vortex au sein de l'espace circulaire, vortex qui viendra effleurer l'extrémité distale de l'écouteur 101. En particulier, la dissymétrie provenant de l'orientation de chaque rainure 308 et de la paroi en demi-cercle 309, améliore l'effet de rotation en écoulement ménageant le vortex ou bien ce tourbillon, dont l'aspiration s'effectue par ladite sortie 311.

On notera que ledit support de dôme 300 peut alors présenter des joints 313 afin d'assurer l'étanchéité avec ladite cartouche 170, en particulier au niveau des sorties 311 avec les trous supérieurs 184 ou les orifices de recyclage 177 ou bien des trous 181.

En somme, l'espace circulaire correspond à la cavité 12.

Dès lors, les moyens d'orientation 14 se présentant sous la forme de saillies, de striures ou de gorges peuvent être ménagés au niveau dudit espace circulaire, changeant l'écoulement du fluide et par conséquent améliorant l'effet vortex.

Par ailleurs, de l'autre côté de l'espace circulaire, en face de la paroi 309, un entonnoir 312 est ménagé assurant le soufflage de l'air chaud. L'entonnoir 312 est ménagé de façon convergente depuis une extrémité ouverte en périphérie de la partie inférieure 303 jusqu'à une extrémité opposée débouchant au sein de l'espace circulaire.

De plus, cette forme en entonnoir réduit la quantité de fluide qui pourrait ne pas être aspirée par le vortex et la sortie 311. Cette forme particulière en entonnoir crée aussi un effet venturi qui accélère l'air chaud soufflé.

A ce titre, lors de la mise en circulation dudit fluide, ce dernier peut être chauffé par des moyens adaptés ou au contact de la température ambiante ou de l'air chauffé par lesdits moyens de chauffage 5.

A ce titre, comme visible sur la figure 5, ledit dispositif de nettoyage 2 comprend des moyens 5 de chauffage de l'air et des moyens 6 de mise en circulation de l'air chauffé au sein d'un circuit 7 débouchant à l'intérieur dudit espace 3.

On notera que lesdits moyens de chauffage 5 peuvent se présenter sous la forme de chauffage de type électrique, tel des résistances électriques 50 au contact de l'air à chauffer et formant alors un échangeur thermique.

De plus, lesdits moyens de mise en circulation peuvent se présenter sous la forme d'une pompe ou d'un ventilateur, constituant des moyens de soufflage de l'air chauffé à une extrémité dudit circuit 7 et d'aspiration de l'air depuis l'extrémité opposée. En particulier, ledit circuit 7 est ouvert au niveau de l'espace 3, par l'intermédiaire d'ouvertures 8 de soufflage et de reprise d'air, de sorte que le soufflage propulse l'air chauffé au sein dudit espace 3, tandis que l'aspiration permet le retour de l'air vers lesdits moyens de mise en circulation 6. Dès lors, ledit espace 3 peut être prévu hermétique, en dehors desdites ouvertures 8, en position de fermeture de l'enceinte du dispositif 2.

Outre la température de chauffage de l'air par des moyens de chauffage 5 est à même dessécher l'atmosphère à l'intérieur de l'enceinte et les prothèses, en particulier les embouts des écouteurs 101.

De manière plus précise, la température de chauffe des moyens de chauffage 4 est prévue de manière à ne pas détériorer les composants électroniques dudit écouteur 101. La température la plus basse permettant le chauffage et le séchage peut être de 40 degrés Celsius. La température la plus haute peut être de 60 degrés Celsius, évitant d'endommager les composants électroniques.

Avantageusement, lors du fonctionnement du dispositif 2 selon l'invention, le liquide est mis en circulation comme évoqué précédemment, pendant une durée déterminée, suffisante pour nettoyer intégralement l'embout de chaque écouteur 101. Un cycle de nettoyage peut s'étaler sur plusieurs minutes, voir plusieurs dizaines de minutes, notamment et approximativement 20 minutes.

Un laps de temps est alors nécessaire pour l'égouttage, à savoir l'écoulement du fluide ou son aspiration vers ledit réservoir 17. Il ne reste alors que des particules du liquide et de l'humidité ambiante au niveau de la cavité 12.

Au cours et/ou après ce nettoyage par humidification d'une partie de l'embout de chaque écouteur 101, puis cette phase d'écoulement, de l'air est chauffée et mis en circulation pour sécher les écouteurs 101. Un cycle de séchage peut s'étaler sur plusieurs minutes, voir plusieurs dizaines de minutes, notamment et approximativement 20 minutes.

Ainsi, cette étape de séchage assure le retrait total de toute humidité ou présence de la moindre particule de fluide ou liquide qui subsisterait suite au nettoyage, évitant tout risque de court-circuit au niveau de l'électronique lors du branchement ou de la remise en marche de la prothèse 1.

De plus, l'enceinte peut prévoir une circulation de l'air chaud soufflé, notamment au travers de l'espace 3 par l'intermédiaire des logements 4, ou bien selon des ouvertures ménagées à l'intérieur de ladite enceinte. Dès lors, une circulation naturelle d'air moins chaud peut s'effectuer du bas vers le haut de l'enceinte, assurant qu'aucune humidité ambiante ne vienne au contact de la prothèse 1, en dehors de l'extrémité des embouts de chaque écouteur 101.

Selon des modes particuliers de réalisation, des moyens de contrôle du chauffage, des mises en circulation de l'air et du liquide, de la durée de nettoyage, peuvent être ajoutés. Ils permettent de définir des cycles de nettoyage, de façon automatique ou paramétrable.

Ainsi, la présente invention assure un nettoyage amélioré par humidification de la partie encrassée de l'embout de chaque écouteur 101, assurant une liquéfaction du cérumen et son aspiration au plus près de chaque écouteur 101, sans détériorer ce dernier.

## Revendications

1. Dispositif (2) de nettoyage d'au moins un appareillage médical auditif comprenant un écouteur (101) constitué d'un embout conformé de manière à être introduit dans le conduit auditif d'une personne, le dispositif (2) de nettoyage comprenant, d'une part, un espace (3) de réception de l'écouteur (101) qui comprend au moins un logement (4) conformé de manière à recevoir l'embout, le logement (4) comprenant en partie basse un orifice (11) conformé de sorte que l'extrémité distale de l'embout dépasse au sein d'une cavité (12) ménagée sous le logement (4), et d'autre part, des moyens de mise en circulation (9) au sein de l'espace (3) d'un liquide de sorte que ce dernier vienne au contact de l'embout, lesdits moyens de mise en circulation (9) dudit liquide débouchant au sein de ladite cavité (12) sous la forme d'au moins une buse (13), la cavité (12) comprenant au moins une sortie (15) d'écoulement ou d'aspiration du liquide, débouchant en vis-à-vis sous chaque orifice (11), **caractérisé en ce que** les moyens de mise en circulation (9) projettent le liquide, afin que le liquide vienne effleurer l'extrémité distale de l'embout, le fond de la cavité (12) comprend des moyens d'orientation (14) du liquide jusqu'à la sortie (15) située en dessous de l'orifice (11) l'orientation, l'évacuation et l'écoulement ou l'aspiration assurant que la cavité (12) ne se remplisse pas de fluide.

2. Dispositif (2) de nettoyage selon la revendication 1, **caractérisé en ce que** lesdits moyens d'orientation (14) se présentent sous la forme de saillies, de striures ou de gorges.

3. Dispositif (2) de nettoyage selon la revendication 2, **caractérisé en ce qu'**il comprend des moyens de chauffage (5) dudit espace (3) par mise en circulation d'air chauffé.

4. Dispositif (2) de nettoyage selon l'une quelconque des revendications 1 ou 2, **caractérisé par le fait qu'**il comprend des moyens de filtration (16) situés en partie inférieure sous ladite cavité (12).

5. Dispositif (2) de nettoyage selon la revendication 3, **caractérisé par le fait que** lesdits moyens de filtration (16) se présentent sous la forme d'une membrane perméable constituée en fibres, mousse ou en un matériau tissé dont l'espacement permet de récupérer les particules de cérumen, liquéfiée ou non, ainsi que les poussières et saletés, tout en laissant passer le liquide.

6. Dispositif (2) selon l'une quelconque des revendications précédentes, **caractérisé par le fait qu'**il comprend une cartouche (170) de forme cylindrique insérée au sein dudit espace (3), ladite cartouche (170) étant subdivisée intérieurement en plusieurs compartiments de stockage (172) et de recyclage (176) délimités par la paroi périphérique dudit cylindre et des parois radiales (174).

7. Dispositif (2) selon la revendication 6, **caractérisé par le fait que** lesdits compartiments de stockage (172) comprennent des orifices (180) ménagés en face inférieure (179) refermés par des opercules (1800), ainsi que des trous (181) ménagés en face supérieure (178), et que lesdits compartiments de recyclage (176) comprennent deux orifices de recyclage (177) ménagés en face supérieure (178) de ladite cartouche (170).

8. Dispositif (2) selon la revendication 7, **caractérisé par le fait que** ladite cartouche (170) comprend deux canaux centraux (183) sous forme cylindrique traversant intérieurement ledit cylindre depuis la face supérieure (178) jusqu'à la face inférieure (179) et débouchant à l'extérieur au niveau réciproquement de trous supérieurs (184) ménagés en face supérieure (178) et de trous inférieurs (185) ménagés en face inférieure (179).

9. Dispositif selon l'une quelconque des revendications précédentes, **caractérisé par le fait qu'**il comprend un support de dôme (300) présentant une section complémentaire à celle de la section de l'espace (3), de manière à venir s'y insérer en partie supérieure et, après insertion, ledit support de dôme (300) étant monté mobile est rotation selon l'axe central dudit espace (3), de manière à être tourné dans le sens des aiguilles d'une montre, ou inversement, dans plusieurs positions de fonctionnement.

10. Dispositif selon la revendication 9, **caractérisé par le fait que** ledit support de dôme (300) comprend une partie supérieure (302) et une partie inférieure (303), cette dernière étant creusée, de manière à y ménager des rainures à l'intérieure desquelles circulera le fluide lors du nettoyage, une ouverture (307) étant ménagée en périphérie et reliée à une bifurcation reliant deux rainures symétriques (308) venant déboucher chacune au droit d'une paroi (309) en arc de cercle déviant de part et d'autre ledit fluide lors de sa circulation jusqu'au niveau de chaque extrémité de chaque paroi (309) vers une butée (310) ménagée à angle droit détournant le fluide vers l'intérieur.

## Patentansprüche

1. Vorrichtung (2) zum Reinigen von mindestens einem medizinischen Hörgerät, umfassend einen Ohrhörer (101), der aus einem Endstück besteht, das gestaltet ist, um in den Gehörgang einer Person eingeführt zu werden, wobei die Vorrichtung (2) zum Reinigen einerseits einen Raum (3) zum Aufnehmen des Ohrhörers (101) umfasst, der mindestens eine Aufnahme (4) umfasst, die ausgestaltet ist, um den Ohrhörer (101) aufzunehmen, wobei die Aufnahme (4) im unteren Teil ein Loch umfasst (11), das so ausgestaltet ist, dass das distale Ende des Endstücks im Inneren eines unter der Aufnahme (4) vorgesehen Hohlraums (12) hineinragt, und andererseits Mittel zum in Umlauf Bringen (9) innerhalb des Raums (3) einer Flüssigkeit, so dass letztere mit dem Endstück in Berührung gelangt, wobei die besagten Mittel zum in Umlauf Bringen (9) der besagten Flüssigkeit in den als wenigestens eine Düse (13) ausgestalteten Hohlraum (12) ausmünden, wobei der Hohlraum (12) mindestens einen Auslass (15) zum Herausstoßen oder Ansaugen der Flüssigkeit umfasst, der gegenüberliegend unter jeder Öffnung (11) ausmündet, **dadurch gekennzeichnet, dass** die Mittel zum in Umlauf Bringen (9) die Flüssigkeit ausstoßen, so dass die Flüssigkeit mit dem distalen Ende des Endstücks in Berührung kommt, wobei der Boden des Hohlraums (12) Mittel (14) umfasst, um die Flüssigkeit zu dem unter der Öffnung (11) befindlichen Auslass (15) zu richten, wobei die Ausrichtung, die Evakuierung und der Umlauf oder das Ansaugen sicherstellen, dass der Hohlraum (12) nicht mit Flüssigkeit gefüllt wird.

2. Vorrichtung (2) zum Reinigen nach Anspruch 1, **dadurch gekennzeichnet, dass** die besagten Ausrichtungsmittel (14) als Vorsprüngen, Streifen oder Rillen ausgestaltet sind.

3. Vorrichtung (2) zum Reinigen nach Anspruch 2, **dadurch gekennzeichnet, dass** sie Mittel zum Heizen (5) des besagten Raums (3) durch in Umlauf Bringen von erhitztem Luft umfasst.

4. Vorrichtung (2) zum Reinigen nach irgendeinem der Ansprüche 1 oder 2, **dadurch gekennzeichnet, dass** sie Filtriermittel (16) umfasst, die sich im unteren Teil unter dem besagten Hohlraum (12) befinden.

5. Vorrichtung (2) zum Reinigen nach Anspruch 3, **dadurch gekennzeichnet, dass** die besagten Filtriermittel (16) als eine durchlässige Membran ausgestaltet sind, die aus Fasern, Schaumstoff oder gewebtem Material besteht, deren Beabstandung es erlaubt, verflüssigte oder nicht verflüssigte Ohrenschmalz-Partikel, sowie Staub und Schmutz zu gewinnen, während die Flüssigkeit durchgelassen wird.

6. Vorrichtung (2) nach irgendeinem der vorhergehenden Ansprüche, **dadurch gekennzeichnet, dass** sie eine in den besagten Raum (3) eingeführte Patrone (170) zylindrischer Form umfasst, wobei die Patrone (170) innen in mehrere Speicher- (172) und Rückfuhrkammern (176) unterteilt ist, die von der Umfangswand des besagten Zylinders und von radialen Wänden (174) begrenzt sind.

7. Vorrichtung (2) nach Anspruch 6, **dadurch gekennzeichnet, dass** die besagten Speicherkammern (172) in der unteren Fläche (179) vorgesehene Löcher (180), die durch Deckel (1800) verschlossen sind, sowie in der oberen Fläche (178) vorgesehene Löcher (181) umfassen, und dass die besagten Rückführkammern (176) zwei in der oberen Fläche (178) der besagten Patrone (170) vorgesehene Rückführlöcher (177) umfassen.

8. Vorrichtung (2) nach Anspruch 7, **dadurch gekennzeichnet, dass** die besagte Patrone (170) zwei zentrale Kanäle (183) zylindrischer Form umfasst, die innen durch den besagten Zylinder von der oberen Fläche (178) bis zur unteren Fläche (179) laufen und außen im Bereich der in der oberen Fläche (178) vorgesehenen oberen Löcher (184) bzw. der in der unteren Fläche (179) vorgesehenen unteren Löcher (185) ausmünden.

9. Vorrichtung nach irgendeinem der vorhergehenden Ansprüche, **dadurch gekennzeichnet, dass** sie einen Domträger (300) umfasst, der einen zum Querschnitt des Raums (3) ergänzenden Querschnitt aufweist, um sich in den oberen Teil einzufügen und wobei der Domträger (300) nach der Einfügung entsprechend der Mittelachse des besagten Raums (3) drehbeweglich gelagert ist, um in Uhrzeigersinn oder umgekehrt in mehrere Betriebspositionen gedreht zu werden.

10. Vorrichtung nach Anspruch 9, **dadurch gekennzeichnet, dass** der Domträger (300) einen oberen Teil (302) und einen unteren Teil (303) umfasst, wobei letzterer ausgehöhlt ist, um dort Nuten vorzusehen, in denen das Fluid während der Reinigung fließt, wobei eine Öffnung (307) in dem Umkreis gebildet und mit einer Verzweigung verbunden ist, die zwei symmetrische Nuten (308) verbindet, die jeweils gegenüber einer kreisbogenförmigen Wand (309) ausmünden, die beiderseits das Fluid während seiner Umlauf bis im Bereich jedes Endes jeder Wand (309) zu einem im rechten Winkel vorgesehenen Anschlag (310) umlenken, der das Fluid nach innen umlenkt.

## Claims

1. Device (2) for cleaning at least one auditory medical device comprising an earphone (101) consisting of an earmold shaped to be inserted into the ear canal of a person, the cleaning device (2) comprising, on the one hand, a space (3) for receiving the earphone (101), which comprises at least one accomodation (4) shaped to receive the earmold, the accomodation (4) comprising in the lower portion an orifice (11) shaped so that the distal end of the earmold protrudes within a cavity (12) arranged under the accomodation (4) and, on the other hand, means for putting into circulation (9) within the space (3) a liquid so that the latter enters into contact with the earmold, said means for putting into circulation (9) said liquid ending into said cavity (12) in the form of at least one nozzle (13), the cavity (12) comprising at least one outlet (15) for discharging or aspirating the liquid, ending in front under each orifice (11), wherein the means for putting into circulation (9) project the liquid, so that the liquid touches the distal end of the earmold, the bottom of the cavity (12) comprises means for directing (14) the liquid to the outlet (15) located under the orifice (11), the orientation, the evacuation and the flowing out or the aspiration ensuring that the cavity (12) does not fill with fluid.

2. Cleaning device (2) according to claim 1, wherein said orientation means (14) are in the form of projections, striations or grooves.

3. Cleaning device (2) according to claim 2, wherein it comprises means for heating (5) said space (3) by putting heated air into circulation.

4. Cleaning device (2) according to any one of claims 1 or 2, wherein it comprises filtering means (16) located in the lower portion under said cavity (12).

5. Cleaning device (2) according to claim 3, wherein said filtering means (16) are in the form of a permeable membrane consisting of fibers, foam or woven material the spacing of which permits to collect the particles of cerumen, whether liquefied or not, as well as dust and dirt, while letting the liquid pass through.

6. Device (2) according to any one of the preceding claims, wherein it comprises a cylindrically shaped cartridge (170) inserted into said space (3), said cartridge (170) being subdivided internally into several storage (172) and recycling (176) compartments delimited by the peripheral wall of said cylinder and radial walls (174).

7. Device (2) according to claim 6, wherein said storage compartments (172) comprise ports (180) formed in the lower face (179) closed by lids (1800), and ports (181) formed in the upper face (178), and said recycling compartments (176) comprise two recycling ports (177) formed in the upper face (178) of said cartridge (170).

8. Device (2) according to claim 7, wherein said cartridge (170) comprises two cylindrically shaped central channels (183) passing internally through said cylinder from the upper face (178) to the lower face (179) and ending outside at the level of the upper ports (184) formed in the upper face (178) or reciprocally the lower ports (185) formed in the lower face (179).

9. Device according to any one of the preceding claims, wherein it comprises a dome support (300) having a cross-section complementary to the cross-section of the space (3), so as to insert into the latter in the upper portion and, after the insertion, said dome support (300) being mounted movable in rotation according to the central axis of said space (3), so as to be rotated clockwise, or inversely, into several operating positions.

10. Device according to claim 9, wherein said dome support (300) comprises an upper portion (302) and a lower portion (303), the latter being hollowed, so as to provide grooves therein, inside which the fluid will circulate during the cleaning, an opening (307) being provided at the periphery and connected to a bifurcation connecting two symmetrical grooves (308) ending each in front of a wall (309) in the form of an arc of a circle deflecting said fluid on both sides during its flowing to the level of each end of each wall (309) towards a stop (310) provided for at right angles and diverting the fluid inwardly.
